# EUROPEAN PATENT APPLICATION

(11) **EP 2 431 051 A1**
(43) Date of publication of application: **21.03.2012**
(21) Application number: 10009656.9
(22) Date of filing: 15.09.2010
(51) Int. Cl.: A61K 38/21, A61P 25/28

(54) **Type-1 interferons for the treatment of multiple sclerosis**

(71) Applicant: Vakzine Projekt Management GmbH, 30625 Hannover (DE)
(72) Inventor: Oertelt, Clemens, 40476 Düsseldorf (DE); Grode, Leander, 38118 Braunschweig (DE)
(74) Representative: von Renesse, Dorothea

(57) **Abstract**

The invention relates to the field of multiple sclerosis. More particularly, this invention relates to the treatment of multiple sclerosis using Type-1 interferon.

## Description

The invention relates to the field of multiple sclerosis. More particularly, this invention relates to the treatment of multiple sclerosis using Type-1 interferons.

Multiple sclerosis ("MS") is a chronic and progressive inflammatory disease of the central nervous system ("CNS"). The disease is characterized by loss of the myelin layer that insulates nerve fibers. The areas of demyelination, or plaques, are inflammatory in nature with infiltration by T and B-lymphocytes and macrophages. The disease which is usually first diagnosed at an age of about 30 years affects approx. 780,000 patients worldwide.

The person with MS can suffer almost any neurological symptom, including changes in sensation such as loss of sensitivity or tingling, pricking or numbness (hypoesthesia and paraesthesia), muscle weakness, muscle spasms, or difficulty in moving; difficulties with coordination and balance (ataxia); problems in speech (dysarthria) or swallowing (dysphagia), visual problems (nystagmus, optic neuritis including phosphenes, or diplopia), fatigue, acute or chronic pain, and bladder and bowel difficulties. Cognitive impairment of varying degrees and emotional symptoms of depression or unstable mood are also common. Uhthoffs phenomenon, an exacerbation of extant symptoms due to an exposure to higher than usual ambient temperatures, and Lhermitte's sign, an electrical sensation that runs down the back when bending the neck, are particularly characteristic of MS although not specific. The main clinical measure of disability progression and symptom severity is the Expanded Disability Status Scale (EDSS). Life expectancy of patients is 5 to 10 years lower to that of the unaffected population.

Patients with multiple sclerosis can be generally categorized into different subgroups as follows. Relapsing remitting multiple sclerosis ("RR-MS") is characterized by periodic exacerbations of MS symptoms followed by periods of remission with complete or near complete recovery. In secondary progressive multiple sclerosis ("SP-MS") there are distinct attacks as in RR-MS, but the intervening recovery is incomplete and there is a progression of disability in the recovery phases. In primary progressive multiple sclerosis ("PP-MS"), there is a rapid deterioration in condition to severe disability with no discernable periods of remission.

The pathogenesis of MS is despite a lot of scientific efforts still unknown. Several causes are discussed. Theories suggest that MS has a rather complex pathological background, namely it is assumed that it results from a combination of genetic, autoimmune, inflammatory and environmental factors. Nevertheless, sound evidence or unambiguous supportive data for an explanation is still missing.

There is currently no therapeutic cure for MS, although a number of drugs or drug candidates are discussed or used to treat the symptoms of the disease or to inhibit or slow down the progression of the disease. Among the approved drugs (e.g. interferon beta-1 a (trade names Avonex, CinnoVex, ReciGen and Rebif), interferon beta-1b (U.S. trade name Betaseron, in Europe and Japan Betaferon), glatiramer acetate (Copaxone), mitoxantrone, and natalizumab (marketed as Tysabri)) interferon-beta is one of the most effective ones. Therapy with Type-1 interferons is well established and considered the first-line therapy in the treatment of RR-MS.

Although there is a long history with the use of interferon beta for MS the mode of action of the drug is still unknown.

The elucidation of the pathogenesis of MS or the mode of action of MS medication is hindered by the fact that MS represents a complex pathophysiology with unclear etiology. This might also be the reason why various drug developments have been ceased or clinical trials with promising candidates have failed.

Type-1 interferons suffer from a relatively short serum half-life and a low tolerability. Currently only approximately 30% of the MS patients benefit from interferon beta therapy. To overcome this drawback extensive efforts were made in the past to develop interferon derivates with improved pharmacokinetic properties, such as for example PEGylated interferon beta (Pepinsky et al. 2001). EP 0 975 761 B1 discloses interferon beta derivatives where hydrophobic amino acids have been replaced by hydrophilic amino acids. It is speculated that these interferon beta derivatives exhibit improved pharmacokinetic properties without losing the efficacy of the unmodified interferon beta. However, evidence that these derivatives are also efficacious in treating MS is outstanding.

Thorsten Ulf Meyer (Ph.D. thesis "Expression, Aufreinigung und Charakterisierung einer Variante des humanen Interferon-beta aus CHO-Zellen mit verbesserten biochemischen und pharmakokinetischen Eigenschaften" University of Hannover, Germany, October 2000) discloses a specific Type-1 interferon, where eight hydrophobic amino acids and one cysteine were replaced by hydrophilic serine moieties. As a result the hydrophobicity was reduced - leading to a better tolerability - and the bioavailability in rabbits was increased up to six-fold when compared to human interferon beta. Accordingly this derivate was shown to exhibit improved pharmacokinetic properties. Notably Meyer has not presented any data on its pharmacodynamic properties. Thus, the efficacy of this drug candidate for the treatment of MS is still unknown.

The objective underlying the invention is to provide a safe and efficacious method for the treatment of MS, in particular to provide a drug substance for use in treating this disease, wherein the drug substance is improved in terms of its tolerability compared to human interferon beta. The drug substance favourably exhibits at least a similar efficacy in treating MS in humans as human interferon beta.

This objective is solved by providing a Type-1 interferon as of claim 1 for the use of multiple sclerosis, in particular the Type-1 interferon as of SEQ ID No 6 or 7. SEQ ID' No. 7 is hereinafter designated as Soluferon.

The Type-1 interferons of the invention are characterized by an at least 90%, more preferably at least 98%, most preferred at least 99% identity to human interferon beta according to SEQ ID No 1 to 4, wherein the amino acids in position 5, 8, 47, 50, 106, 111, 116 and 120 (according to the numbering of SEQ ID No 1 to 4) are substituted with a non-hydrophobic amino acid. The non-hydrophobic amino acids are preferably selected from the group consisting of the hydrophilic, basic, acidic or polar amino acids arginine, asparagine, aspartate, glutamate, glutamine, histidine, lysine, methionine, proline serine, threonine, tryptophane and tyrosine. Most preferred they are substituted with serine. Furthermore at position 17 (according to the numbering of SEQ ID No 1 to 4) the Type-1 interferon s of the invention have in position 17 an amino acid other than cysteine, in order to prevent inter- or intramolecular cysteine bridge mismatches. Preferably cysteine at position 17 is substituted with serine.

A preferred Type-1 interferon as of the invention is depicted in SEQ ID No 6, which encompasses the amino acid positions 5, 8, 47, 50, 106, 111, 116 and 120 substituted as described above. In one embodiment of the invention the substitutions each are serine.

In a particular preferred embodiment the Type-1 interferon as of the invention has the sequence of SEQ ID No 7. This Type-1 interferon is denominated as Soluferon. Its sequence corresponds to SEQ ID No 6 with all amino acids in position 5, 8, 17, 47, 50, 106, 111, 116 and 120 (according to the numbering of SEQ ID No 1 to 4) substituted with serine and furthermore having a deletion of one amino acid at the N terminus. Soluferon is the most preferred embodiment of the invention. It has to be noted, that due to the N-terminal deletion of the amino acid methionine, the substitutions in SEQ ID No 7 are now at amino acid positions 4, 7, 16, 46, 49, 105, 110, 115 and 119 of SEQ ID No. 7 (corresponding to amino acid positions 5, 8, 17, 47, 50, 106, 111, 116 and 120 of SEQ ID 1-4 or No 6).

The invention comprises also the use of microheterogeneous forms of the interferon beta derivatives disclosed in here. Microheterogeneous forms of the interferon beta derivatives encompass in particular interferon beta molecules with one, two or three deletions at the N and/or C-terminus, e.g. one amino acid deletion at the N-terminus.

The inventors found that the Type-1 interferons of the invention, in particular Soluferon, show similar pharmacodynamic properties to commercially available interferon beta (e.g. Rebif). In particular they found that the treatment of Cynomolgus monkeys with these Type-1 interferons of the invention - as exemplified with Soluferon - leads to increased plasma levels of neopterin and 2',5'-oligoadenylate-synthetase (2',5'OAS). In this model the commercially available interferon beta (e.g. Rebif) showed a similar increase of neopterin and 2',5'OAS. Therewith, the inventors could demonstrate that the Type-1 interferons of the invention, in particular Soluferon, show similar pharmacodynamic properties to the established MS drug Rebif.

Neopterin serves as an activation marker of the cellular immune system. 2',5' OAS is induced by interferon which lead to IFN-gamma-induced apoptosis. 2',5' OAS and neopterin are accepted as significant parameter for monitoring parenterally administered interferon beta and its pharmacodynamic effects.

Furthermore the inventors were able to demonstrate the safety of Soluferon since it was tolerated well in terms of electrocardiography (ECG), body temperature and general clinical parameters.

In addition, an *in silico* analysis of the amino acid sequence indicated reduced antigenicity of Soluferon as compared to the marketed interferon beta.

Hence the inventors could demonstrate for the first time that the higher solubility and bioavailability of the Type-1 interferons of the invention (such as Soluferon) does not compromise safety and its biological activity with regard to treatment of MS.

The Type-1 interferon s of the invention can be provided with the common knowledge of the skilled person, e.g. based on the nucleotide sequence of the human interferon beta using conventional methods of directed mutagenesis.

Recombinant interferon has been cloned and expressed in *Escherichia coli* by several groups, for example, Streuli et al. 1980 (Science 209: 1343-1349). The purification of recombinant interferons has been described by several groups using a combination of chromatographic steps such as ammonium sulfate precipitation, dye 55 affinity chromatography, ion exchange and gel filtration, for example, as described in Weissmann 1982, Phil. Trans. R. Soc. Lond. B, 299:7-28). An alternative approach for purifying recombinant interferon employs immunoaffinity chromatography with an immobilized antibody (P.P. Trotta et al. 1987, Developments in Industrial Microbiology 72, Elsevier, Amsterdam: 53-64). For a review of available purification schemes used for recombinant interferons, see T.L. Nagabhushan and P.P. Trotta, Ullmann's Encyclopedia of Industrial Chemistry. A14, VCH, Weinheim, Federal Republic of Germany (1989) 372-374. Preferably, the interferon used is purified by a conventional purification process described in Ullmann's Encyclopedia of Industrial Chemistry. A14, VCH, Weinheim, Federal Republic of Germany (1989) 372-374 followed by reversed phase high performance chromatography.

The Type-1 interferon used according to the invention is preferably recombinantly produced by genetic engineering techniques. The Type-1 interferon may be produced by prokaryotic, e.g. E. coli cells, or eukaryotic, e.g. Chinese hamster ovary (CHO) cells. Preferably, a Type-1 interferon produced by CHO cells is used according to the invention. A method to provide Soluferon as a specific Type-1 interferon in CHO cells is described in detail in Meyer TU (PhD thesis, University of Hannover, Germany, October 2000).

The pharmaceutical compositions prepared according to the invention comprise the Type-1 interferon together with a pharmaceutically acceptable carrier. The term "pharmaceutically acceptable" includes any carrier which does not interfere with the effectiveness of the biological activity of the Type-1 interferon and which is not toxic to the host to which it is administered.

As one embodiment of the invention, the compositions are prepared using a Type-1 interferon stabilized with human serum albumin. For this purpose, a preparation of the Type-1 interferon produced in CHO cells, is purified and then lyophilised with human serum albumin in vials.

In one aspect of the invention the Type-1 interferon is nonglycosylated. However, in a preferred aspect of the invention a glycosylated form of Type-1 interferon is used, more preferably a mono-glycosylated form.

The glycosylated Type-1 interferon can be N-glycosylated and/or O-glycosylated. In a preferred aspect the glycosylated form is N-glycosylated.

The most preferred embodiment of the invention, namely Soluferon, can be used either glycosylated or non,glycosylated. Preferably a glycosylated form of Soluferon is used, which is more preferably a mono-glycosylated form of Soluferon.

The Type-1 interferons of the invention (in particular Soluferon) can be preferably used to treat MS patients within the four standardized MS subtypes as defined in 1996 by the United States National Multiple Sclerosis Society:
1. Relapsing remitting MS, (with benign MS)
2. Secondary progressive MS,
3. Primary progressive MS, and
4. Progressive relapsing MS.

The **relapsing-remitting** subtype is characterized by unpredictable relapses followed by periods of months to years of relative quiet (remission) with no new signs of disease activity: Deficits suffered during attacks may either resolve or leave sequelae, the latter being more common as a function of time. This describes the initial course of 85-90% of individuals with MS.

**Secondary progressive MS** (sometimes called "galloping MS") describes around 65 % of those with an initial relapsing-remitting MS, who then begin to have progressive neurologic decline between acute attacks without any definite periods of remission. Occasional relapses and minor remissions may appear. The median time between disease onset and conversion from relapsing-remitting to secondary progressive MS is 19 years.

The **primary progressive subtype** describes the approximately 10-15% of individuals who never have remission after their initial MS symptoms. It is characterized by progression of disability from onset, with no, or only occasional and minor, remissions and improvements. The age of onset for the primary progressive subtype is later than for the relapsing-remitting, but similar to mean age of progression between the relapsing-remitting and the secondary progressive. In both cases it is around 40 years of age.

**Progressive relapsing MS** describes those individuals who, from onset, have a steady neurologic decline but also suffer clear superimposed attacks. This is the least common of all subtypes.

In a further aspect of the invention the Type-1 interferon could be used to treat also patients with a clinically isolated syndrome (CIS). The relapsing-remitting subtype usually begins with CIS. In CIS, a patient has an attack suggestive of demyelination, but does not fulfill the criteria for MS. However, only 30 to 70% of persons experiencing CIS later develop MS.

In another aspect of the invention the Type-1 interferon could also be used to treat patients with the so called "benign MS". In benign MS; the deficits always resolve between attacks, although patients will still accrue some degree of disability in the long term.

In a still further aspect of the invention the Type-1 interferon could also be used to treat atypical variants of MS with non-standard behaviour which include Devic's disease, Balo concentric sclerosis, Schilder's diffuse sclerosis and Marburg MS.

In one aspect of the invention the Type-1 interferon can be used to treat patients in whom the existing interferon beta brands exhibit poor tolerability and/or are non responders for the existing interferon beta brands.

In a further aspect of the invention the Type-1 interferon can be used to treat patients that developed interferon beta-induced neutralizing antibodies (NAb) as result of an interferon-beta therapy. During interferon beta therapy, the presence of NAb reduces or abolished bioavailability in a relevant percentage of patients.

In one aspect of the invention the Type-1 interferon can be used to treat MS patients to attenuate the severity of the disability or to delay a worsening of the disability as assessed by EDDS. The Expanded Disability Status Scale (EDSS) is a method of quantifying disability in multiple sclerosis

The EDSS quantifies disability in eight Functional Systems (FS) and allows neurologists to assign a Functional System Score (FSS) in each of these. The Functional Systems are: 1.) pyramidal, 2.) cerebellar, 3.) brainstem, 4.) sensory, 5.) bowel and bladder, 6.) visual, 7.) cerebral, and 8.) other.

EDSS steps 1.0 to 4.5 refer to people with MS who are fully ambulatory. EDSS steps 5.0 to 9.5 are defined by the impairment to ambulation. The clinical symptoms which are used to define the scores are given in the following table:

| **Score** | **Symptoms** |
|---|---|
| 0.0 | Normal neurological examination |
| 1.0 | No disability, minimal signs in one FS |
| 1.5 | No disability, minimal signs in more than one FS |
| 2.0 | Minimal disability in one FS |
| 2.5 | Mild disability in one FS or minimal disability in two FS |
| 3.0 | Moderate disability in one FS, or mild disability in three or four FS. Fully ambulatory |
| 3.5 | Fully ambulatory but with moderate disability in one FS and more than minimal disability in several others |
| 4.0 | Fully ambulatory without aid, self-sufficient, up and about some 12 hours a day despite relatively severe disability; able to walk without aid or rest some 500 meters |
| 4.5 | Fully ambulatory without aid, up and about much of the day, able to work a full day, may otherwise have some limitation of full activity or require minimal assistance; characterized by relatively severe disability; able to walk without aid or rest some 300 meters. |
| 5.0 | Ambulatory without aid or rest for about 200 meters; disability severe enough to impair full daily activities (work a full day without special provisions) |
| 5.5 | Ambulatory without aid or rest for about 100 meters; disability severe enough to preclude full daily activities |
| 6.0 | Intermittent or unilateral constant assistance (cane, crutch, brace) required to walk about 100 meters with or without resting |
| 6.5 | Constant bilateral assistance (canes, crutches, braces) required to walk about 20 meters without resting |
| 7.0 | Unable to walk beyond approximately five meters even with aid, essentially restricted to wheelchair; wheels self in standard wheelchair and transfers alone; up and about in wheelchair some 12 hours a day |
| 7.5 | Unable to take more than a few steps; restricted to wheelchair; may need aid in transfer; wheels self but cannot carry on in standard wheelchair a full day; May require motorized wheelchair |
| 8.0 | Essentially restricted to bed or chair or perambulated in wheelchair, but may be out of bed itself much of the day; retains many self-care functions; generally has effective use of arms |
| 8.5 | Essentially restricted to bed much of day; has some effective use of arms retains some self care functions |
| 9.0 | Confined to bed; can still communicate and eat |
| 9.5 | Totally helpless bed patient; unable to communicate effectively or eat/swallow |
| 10.0 | Death due to MS |

As a further method to assess the therapeutic effect of the Type-1 interferon in MS treatment is the T2-weighted or gadolinium (Gd)-enhanced T1-weighted magnetic resonance imaging (MRI) which is used to monitor the disease activity by counting "active" plaques in the CNS.

### Definitions

A "Type-1 interferon" according to the invention is a protein or peptide that binds to and activates the interferon-alpha receptor (IFNAR) comprising of the IFNAR1 and the IFNAR2 chain. Preferably the activation of the interferon-alpha receptor leads to activation of the JAK-STAT signal pathway involving the Janus kinase JAK and the "Signal Transducer and Activator of Transcription" (STAT). More preferably the Type-1 interferon of the invention exhibits antiviral activity as measured in the antiviral activity assay (AVA) which is described by Meyer TU (PhD thesis University of Hannover, Germany, October 2000, page 54-55).

Soluferon is a of the human beta interferon as depicted in SEQ ID No.7, whereas the amino acid positions 5, 8, 17, 47, 50, 106, 111, 116 and 120 are substituted with serine and furthermore the N terminal amino acid methionine is deleted. The amino acid sequence of Soluferon is given in SEQ ID NO 7. Soluferon is under development at Vakzine Projekt Management GmbH, Hannover, Germany. Soluferon is a registered trademark.

The term "treating" or "treatment" refers to any medical measure for preventing, reducing, mitigating or curing physiological disorders within a mammal, in particular a human, in the need thereof.

The term "microheterogeneous forms" as used herein refers to a single gene product, which is a protein produced from a single gene unit of DNA, which is structurally modified following translation. These structural modifications, however, do not result in any significant alterations of the activity of the protein. The modifications may take place either in vivo or during the isolation and purification process. In vivo modification may result in, but is not limited to, acetylation at the N-terminus, amidation at the C-terminus, proteolysis, glycosylation, or phosphorylation. Proteolysis may include exoproteolysis wherein one or more terminal amino acids are sequentially, enzymatically cleaved to produce microheterogeneous forms which have fewer amino acids than the original gene product. Proteolysis may also include endoproteolytic modification that results from the action of endoproteases which cleave the peptide at specific locations within the amino acid sequence. Similar modifications can occur during the purification process which may result in the production of microheterogeneous forms. The most common modification occur during purification is proteolysis.

The term "recombinant" is used herein to describe altered or manipulated nucleic acids, nucleic acids isolated from the environment in which they naturally occur, host cells transfected with or otherwise manipulated to contain exogenous nucleic acids, or proteins expressed non-naturally through manipulation of isolated DNA and transformation of host cells. "Recombinant" is a term that specifically encompasses DNA molecules which have been constructed in vitro using genetic engineering techniques, and use of the term "recombinant" as an adjective to describe a molecule, construct, vector, cell, protein, polypeptide, or polynucleotide specifically excludes naturally occurring such molecules, constructs, vectors, cells, proteins, polypeptides, or polynucleotides. In particular, a "recombinant protein" for the purposes of the present invention is a protein that is expressed by a host cell which has been manipulated by incorporating at least one genetic element that was not naturally occurring in the host cell prior to expression of the protein. A protein, the coding sequence for which has been artificially incorporated into a host cell made capable of expressing the protein, for example by being transfected with an expression vector including the coding sequence of the protein, is a "recombinant protein" once expressed by the host cell.

The term "non-hydrophobic amino acids" in the context of the present invention refers to all amino acids except glycine, alanine, valine, leucine and isoleucine. Non-hydrophobic amino acids include all amino acids which do not possess an aliphatic side chain. Furthermore all amino acids which show a reduced hydrophobicity as compared to the group consisting of glycine, alanine, valine, leucine and isoleucine are belonging to the group of non-hydrophobic amino acids. In the context of the present invention the non-hydrophobic amino acids also encompass non-natural amino-acids.

As used herein, the term "non-natural amino acid" refers to an organic compound that is a congener of a natural amino acid in that it has a structure similar to a natural amino acid so that it mimics the structure and reactivity of a natural amino acid. The non-natural amino acid as defined herein generally increases or enhances the properties of a peptide (e.g., selectivity, stability) when the non-natural amino acid is either substituted for a natural amino acid unit of a peptide or otherwise incorporated into a peptide. Examples for non-natural amino acids are p-methoxyphenylalanine (pMpa), p-acetylphenylalanine (pApa), p-benzoylphenylalanine (pBpa), p-iodophenylalanine (plpa), p-azidophenylalanine (pAzpa), 7-propargyloxyphenylalanine (pPpa), α-aminocaprylic acid, ortho-nitrobenzylcysteine (o-NBC), 1,5-dansylalanine and ortho-nitrobenzylserine (o-NBS), azetidine-2-carboxylic acid, 3,4-dehydroproline, perthiaproline, canavanine, ethionine, norleucine, selenomethionine, aminohexanoic acid, telluromethionine, homoallylglycine, homopropargylglycine, ornithine, taurine, selenocysteine, 3-hydroxyproline, 4-hydroxyproline, hydroxylysine, allo-hydroxylysine, N-ethyl asparagine, N-ethyl lysine, desmosine, isodesmosine, sarcosine, norvaline and pyrrolysine.

### EXAMPLES

### 1. Pharmacokinetics and Pharmacodynamics Study in Cynomolgus monkeys

### 1.1 Purpose

The study was conducted for several reasons. First, it was designed to show general safety of Soluferon. Second, pharmacokinetics of Soluferon was analyzed by means of an antiviral assay (AVA). Third, pharmacodynamics of Soluferon were analyzed based on the induction of neopterin and 2',5'-Oligoadenylate synthetase (2',5'OAS). Neopterin generally serves as an activation marker of the cellular immune system. 2',5'OAS is induced by interferon, which can lead to IFN-gamma-induced apoptosis. Neopterin and 2',5'OAS are accepted as significant parameters for monitoring parenterally administered Interferon beta. Commercially available IFNs approved for therapy of MS had shown effects on these pharmacodynamic parameters. Fourth, the potential induction of neutralizing antibodies (NAb) was analyzed which is reported in Example II (Module Antigenicity). Fifth, single application of Soluferon was compared to a seven day repeated administration.

### 1.2 Experimental Procedure

The study comprised a total of 24 Cynomolgus macaques in 4 groups. Each group consisted of 3 male and 3 female animals. Each animal was administered with a different vehicle. Then, 72 hours after administration of the vehicle, each six-animal group was administered with one of the test or reference items. The following doses were administered.

| | |
|---|---|
| Group I: | 11 µg Soluferon-(with HSA) / animal - single s. c. |
| Group II: | 44 µg Soluferon (with HSA) / animal - single s. c. |
| | 38 µg Soluferon (without HSA) / animal - repeated s. c. injection for 7 days, once per day |
| Group III: | 44 µg Rebif / animal - single s. c. |
| Group IV: | 110 µg Soluferon (with HSA) animal - single s. c. |

| Single Dose Phase | |
|---|---|
| PK: | plasma concentration at 0; 0.5; 1; 3; 6; 9; 12; 24; 48; 72; 96h post dose. |
| PD: | Neopterin and 2',5'-OAS serum concentration at 0; 3; 6; 9; 12; 24; 48; 72; 96h. |
| NAb: | neutralizing antibodies on days 0 and 13. |

| Repeated Dose Phase | |
|---|---|
| PK: | plasma concentration at 0; 0.5; 1; 3; 6; 9; 12h after first dose; predose day 2-7 and 0.5; 1; 3; 6; 9; 12; 24; 48; 72; 96 and 120h after last dose day 7. |
| PD: | Neopterin and 2',5'-OAS serum concentration at 0; 3; 6; 9; 12; 24; 48; 72; 96; 120; 144; 147; 150; 153; 156; 168; 192; 216; 240h after first dose. |
| NAb: | neutralizing antibodies on days 0, 13, 20 and 27. |

Plasma levels of interferon betaand Soluferon were determined as antiviral activity in vitro. Serum levels of neopterin was measured using ELISA and serum 2',5'-OAS activity using a radio immuno assay (RIA) by quality certified laboratories in Germany and Switzerland. Evaluation of the presence of neutralizing antibodies (NAb) to Soluferon was done by means of a cellular assay utilizing WISH cells. Exposure of the cells to Vesicular Stomatitis Virus (VSV) will induce cytopathic effects. Type-1 interferons exert a protective effect, which will be reduced in the presence of NAbs.

### 1.3 Results

### 1.3.1 General safety

There were no differences between the groups with regard to body weight and clinical parameters. There were no adverse events. Body temperature was not affected in animals which received up to 44 µg Soluferon. There was a non-significant transient increase of mean body temperature by 0.2° C in the highest dose group (110 µg Soluferon). The mild but significant increase of the body temperature started approximately 3h after administration and lasted for about 6h. However, this was still within the range of body temperatures found in the vehicle group (range: 38.5° - 39°C). In some preselected (2 animals per group) animals of each group electrocardiograms (ECGs) were performed. The ECGs were neither affected by Soluferon nor by Rebif. No abnormal haematological or biochemical parameters were found. Neither was there any effect induced by vehicle with or without HSA.

### 1.3.2 Pharmacokinetics of Soluferon and Rebif

Disposition to the three tested single doses of Soluferon 11, 44 and 110 µg, to repeated doses of 38 µg Soluferon and to a single Rebif dose of 44 µg was confirmed. The systemic exposure to Soluferon was dependent from the administered dose of Soluferon. There was dose dependence for Cmax and AUC between the three administered doses of Soluferon and a clear increase in systemic exposure in terms of mean AUC(0→t) of Soluferon, obtained when increasing the subcutaneous dose of Soluferon from 11 µg to 110 µg.

| | |
|---|---|
| AUC₀₋₂₄ₕ Rebif 44 µg: | 29283 U^{.}h d^{.}L-1 |
| AUC₀₋₂₄ₕ Soluferon 11 µg: | 111451 U·h d·L-1 |
| AUC₀₋₂₄ₕ Soluferon 44 µg: | 508462 U·h d·L-1 |
| AUC₀₋₂₄ₕ Soluferon 110 µg: | 1843077 U.h d·L-1 |

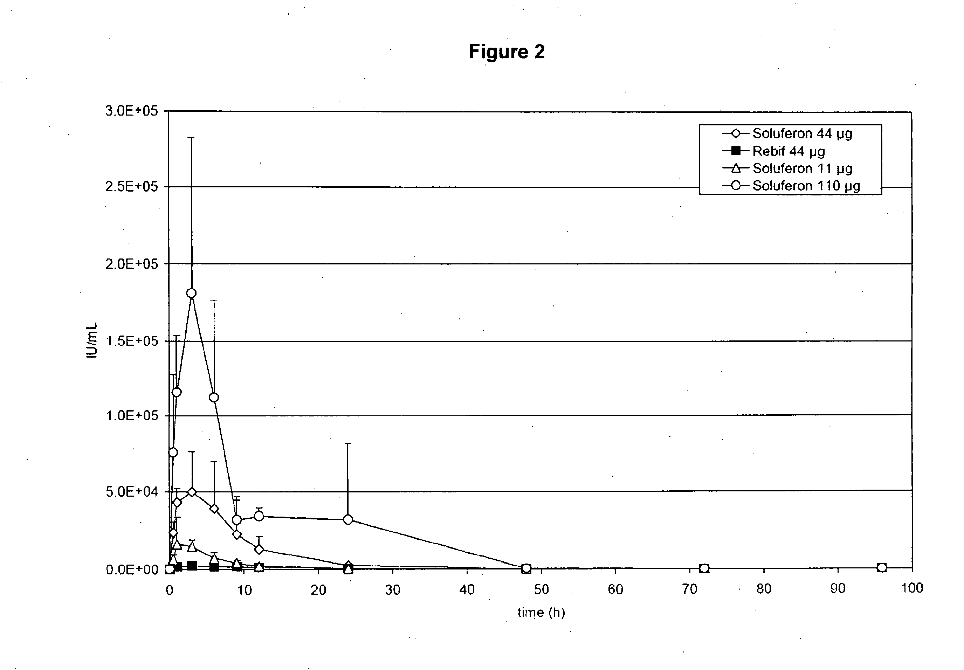

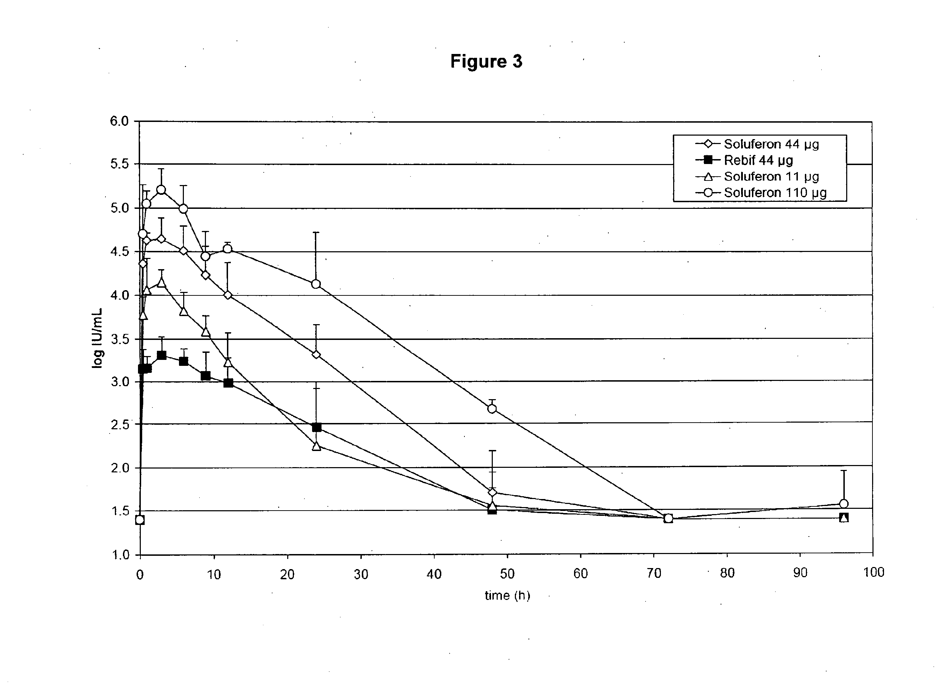

The exposure (AUC) after the administration of Soluferon at the dose of 44 µg was approximately 17-fold higher than that reached after the administration of Rebif at the same dose, confirming the increased bioavailability of Soluferon. Fig. 2 shows the mean antiviral activity as linear data and Fig. 3 as log transformed data.

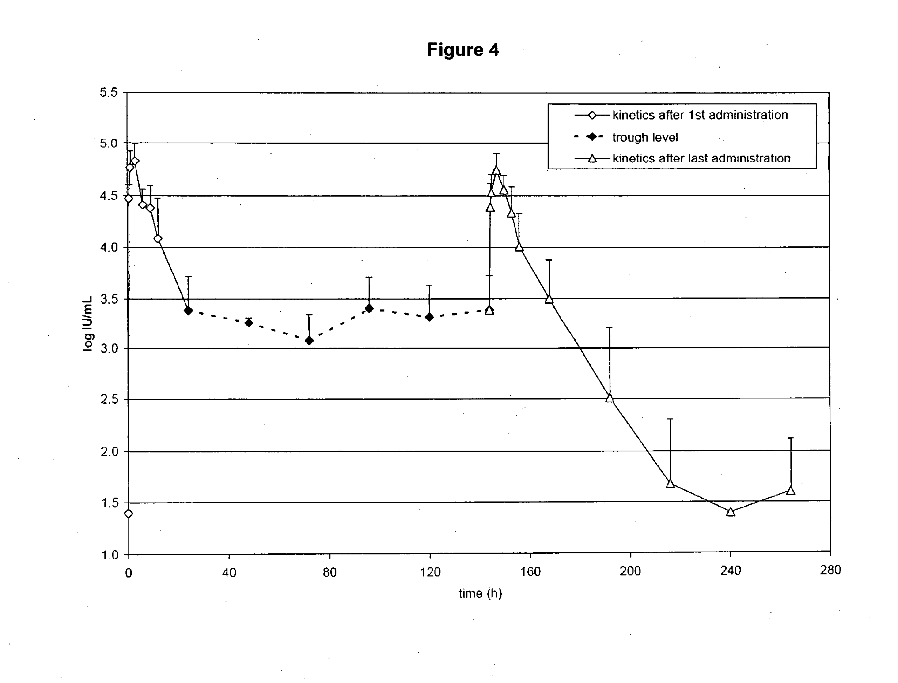

The results did not suggest any influence of the sex on drug exposure after the administration of Soluferon at the three tested doses and the reference item Rebif. After multiple administrations of 38 µg Soluferon during 7 days (Fig. 4), the extent of exposure to Soluferon did not seem to change between the 1 st day and the 7th day of administration. The AUC(0→24h) obtained for Soluferon on days 1 and 7 was similar and confirmed that there was neither accumulation nor a tachyphylaxis of Soluferon. Even 48h after the last administration of Soluferon there was still quantifiable Soluferon bioactivity in the plasma. (Fig. 3).

### 1.3.3 Pharmacodynamic results: Neopterin production and 2',5' OAS activity

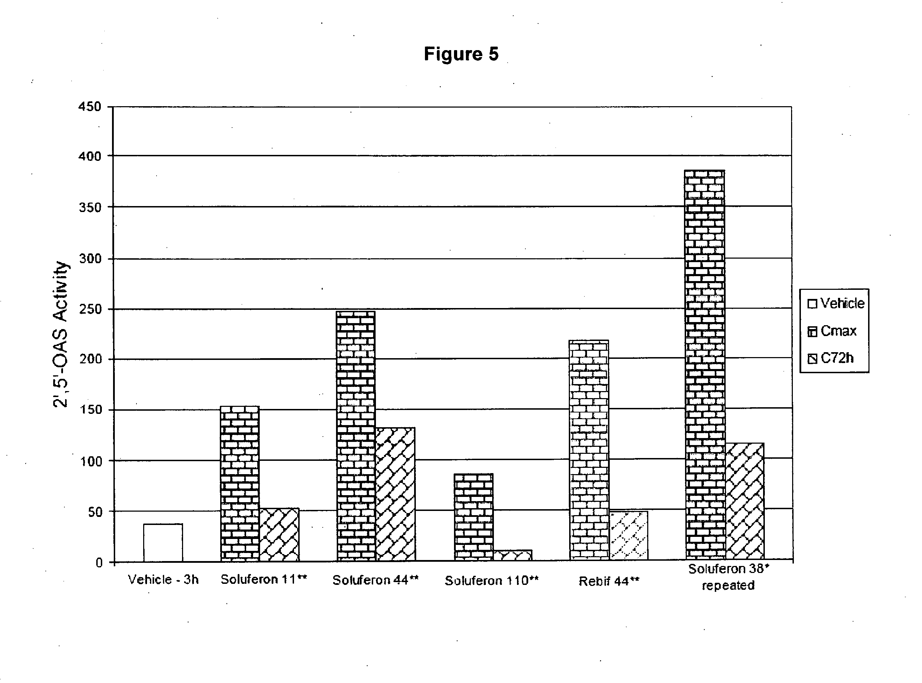

Neopterin and 2',5'-OAS (Fig. 5 and Table 1) were induced after administration of each single dose of Soluferon. There was no induction of neopterin or 2',5'-OAS activity after administration of vehicle with or without HSA above background levels.

The production of neopterin increased as the Soluferon dose increased and exceeded the neopterin level in the vehicle controls even 72h after Soluferon application. Maximum concentrations were achieved approximately 24 hours after the administration. The production of neopterin after the administration of Soluferon at the dose of 44 µg was lower than that reached after the administration of Rebif.

The production of neopterin (as assessed using AUC of neopterin) did not seem to change between the 1st day and the 7th day of administration, confirming the stability-response during a continuous treatment of one week.

**Table 1: AUC-data of the 2',5'-OAS activity after repeated or single application of Soluferon (38 µg or 44 µg) in comparison to vehicle and Rebif (44 µg). Both AUC 0→24h and AUC 0→72h are shown.**

| Substance and Dose | Parameter | Dosing regimen | AUC |
|---|---|---|---|
| Soluferon 38 µg | AUC 0→4h | Vehicle | 2101 pmol x h x dL⁻¹ |
| | AUC 0→24h | day 1 | 5235 pmol x h x dL⁻¹ |
| | AUC 0→24h | day 7 | 5957 pmol x h x dL⁻¹ |
| Soluferon 44 µg/38 µg | AUC 0→72h | Single Dose (44 µg) | 11398 pmol x h x dL⁻¹ |
| | AUC 0→72h | day 7 after repeated dose (38 µg) | 13560 pmol x h x dL-¹ |
| | AUC 0→72h | Vehicle | 5660 pmol x h x dL⁻¹ |
| Rebif 44 µg | AUC 0→72h | Single Dose | 9266 pmol x h x dL⁻¹ |

2',5'-OAS activity (Fig. 5) increased in terms of mean AUC(0→72h) when the dose of Soluferon was increased from 11 µg to 44 µg as compared to the vehicle control. The increase of 2',5'-OAS activity was significantly lower after administration of the high dose of 110 µg as compared to the one after administration of 11 µg and 44 µg. Maximum concentrations were achieved after approximately 12 hours and decreased until 96 hours. The 2',5'-OAS activation after administration of 44 µg Rebif was in between the one induced by 11 µg and 44 µg Soluferon. Table 2 shows the 2',5'-OAS activity at maximum and 72h after administration of a single dose. Furthermore, it shows 2',5'-OAS activity after the last administration of Soluferon as part of the repeated dose regimen. Multiple administrations of 38 µg Soluferon during 7 days, increased the 2',5'-OAS activity as compared to the one after single administration of Soluferon. (Table 2).

**Table 2: 2',5'-OAS serum activity. Medians: N=6 per group**

| Substance | Dose | Remark | Median 2,5-OASₘₐₓ | Median 2.5-OAS₇₂ₕ |
|---|---|---|---|---|
| Rebif | 44 µg | single s. c. | 218 pmol/dl | 48 pmol/dl |
| Soluferon with HSA | 110 µg | single s. c. | 86 pmol/dl | 11 pmol/dl |
| Soluferon with HSA | 44 µg | single s. c. | 247 pmol/dl | 132 pmol/dl |
| Soluferon with HSA | 11 µg | single s. c. | 153 pmol/dl | 52 pmol/dl |
| Soluferon without HSA | 38 µg | Repeated, after last administration | 386 pmol/dl | 115 pmol/dl |

| | | | | |
|---|---|---|---|---|
| *: maximum 2',5'-OAS activity; **: 2',5'-OAS activity 72h after application. | | | | |

### 1.4 Conclusion

Soluferon induces neopterin production and increases 2',5'-OAS-activity including a dose-response relationship. Relative bioavailability of Soluferon is about 17-fold higher than that of Rebif. Soluferon was tolerated well in terms of ECG, body temperature and general clinical parameters..

### 2. In silico analysis of antigenicity

Improved solubility may translate into lower antigenicity. Due to the reduced aggregation potential of the protein the antibody induction is expected to be reduced as well. Consequently, theoretical epitope analysis utilizing a specific algorithm has revealed a significantly lower antigenic potential of Soluferon as compared to natural interferon beta (Mollenkopf et al, 2004). The analysis is based on the MHC I and MHC II class binding capacity of Soluferon-derived peptides (fragments), especially in those regions where amino acids have been replaced. The algorithmic analysis of natural human interferon beta led to an overall score of 116.8 and a MHC binding score of 97.3. The scores of Soluferon were substantially lower and came to 90.9 overall and to 71.8 for binding.

### 3. Animal model to investigate MS drug candidates

### EAE Study in rats

Experimental Allergic Encephalitis (EAE) exhibits certain features of the histopathology and neurobiology of multiple sclerosis. EAE induced in laboratory animals is a tool to investigate emerging therapeutic agents. By using EAE models it is possible to understand the mechanisms of T-cell mediated immune damage of the CNS, and the associated factor to cascade of innate immunity.

### Animals

Female BN rats 8-10 weeks of age can be used in the experiments. They have to be kept under environmentally controlled conditions without the presence of pathogens. All experiments that involve animal use have to be performed in compliance with the relevant laws and institutional guidelines.

### lmmunogens

Several immunogens can be used to induce the allergic encephalitis such as proteolipid protein (PLP), myelin basic protein (MBP), myelin-oligodendrocyte-glycoprotein (MOG), myelin-associated glycoprotein (MAG), 2',3'-cyclic nucleotide 3' phosphodiesterase (CNPase), S100 beta protein, myelin-associated oligodendrocytic basic protein (MOBP), oligodendrocyte-specific glycoprotein (OSPG) or alphaB crystalline. Immunogenic protein fragments of the aforementioned proteins can also be used. Particularly rrMOG^{Igd}, which corresponds to the N-terminal sequence of rat MOG (amino acids 1-125) can be used as immunogen. It can be expressed in *E*. *coli* and purified to homogeneity by chelate chromatography, following dialysis of the purified protein in 6 M urea against PBS to obtain a preparation to be stored at -20°C.

### Induction and evaluation of EAE

After anesthesia the rats are typically injected intradermally at the base of the tail with an inoculum, containing the immunogen emulsified with an adjuvans such as complete Freund's adjuvant (CFA) containing further heat-inactivated mycobacterium tuberculosis. Rats were scored for clinical signs of EAE and weighed daily. The signs were scored as follows: grade 1, tail weakness or tail paralysis; grade 2, hindleg paraparesis or hemiparesis; grade 3, hindleg paralysis or hemiparalysis; grade 4, complete paralysis (tetraplegy), moribund state, or death.

The general time course of the experiment is as follows: electrophysiological measurements were performed 14-30 d after immunization and 48-72 hr after the onset of clinical symptoms. Immediately after the end of the recording, animals received an overdose of chloral hydrate. Brains and spinal cords were removed for immunohistochemistry and histological evaluation.

### Sequence legends

- **SEQ ID No. 1:**: Amino acid sequence of mature human interferon beta (Accession NP_002167.1)
- **SEQ ID No. 2:**: Amino acid sequence of mature human interferon beta (Accession number CAA29339)
- **SEQ ID No. 3:**: Amino acid sequence of mature human interferon beta (Accession number AAA72588)
- **SEQ ID No. 4:**: Amino acid sequence of mature human interferon beta (Accession number AAC41702)
- **SEQ ID No. 5:**: Amino acid sequence of mature human interferon beta (SEQ ID No 1) with amino acid exchanges at positions 5, 8, 17, 47, 50, 106, 111, 116 and 120 (designated as Xaa).
- **SEQ ID No. 6:**: Amino acid sequence of mature human interferon beta (SEQ ID No 1) with amino acid at positions 5, 8, 17, 47, 50, 106, 111, 116 and 120 exchanged by serine
- **SEQ ID No. 7:**: Amino acid sequence of Soluferon

### Figure Legends

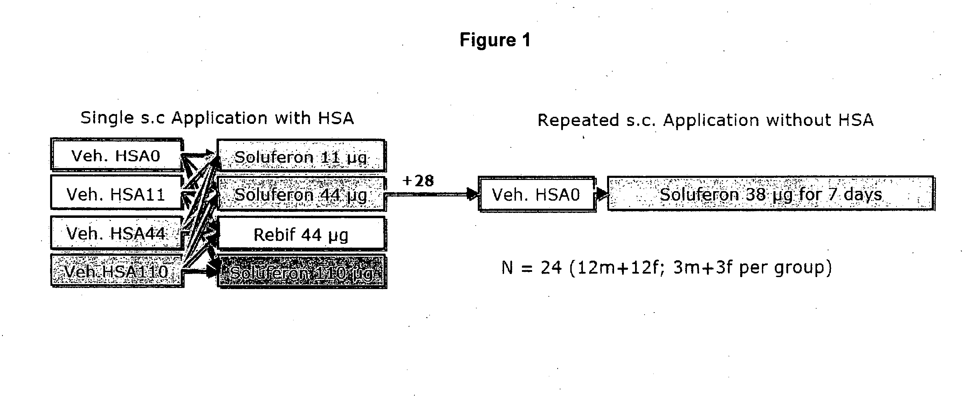

- **Figure 1:**: Schematic outline of the dosing strategy for the PK/PD study in Cynomolgus monkeys testing Soluferon in a concentration of 0, 11, 44, and 110 µg. Human serum albumin (HSA) as vehicle was applied in a concentration of 0, 11, 44 and 110 µg (= HSA0, HSA11, HSA44 and HSA110).
- **Figure 2:**: Antiviral activity (linear data) in Cynomolgus plasma after single application of Soluferon (11 µg, 44 µg or 110 µg) with HSA and Rebif (44 µg), respectively. Mean and standard deviation of the linear data, N=6 per group.
- **Figure 3:**: Antiviral activity (log transformed data) in Cynomolgus plasma after single application of Soluferon (11 µg, 44 µg or 110 µg) with HSA and Rebif (44 µg), respectively. Mean and standard deviation of the log transformed data, N=6 per group.
- **Figure 4:**: Antiviral activity (log transformed data) in Cynomolgus plasma after repeated application of Soluferon without HSA (38 µg). Administration was every 24 hours for a period of one week. Mean and standard deviation of the log transformed data, N=6 per group. During the phase of repeated application plasma samples were analyzed once a day only (black diamonds). Hence the peak levels on days 2 through 6 were not assessed (dotted line).
- **Figure 5:**: Stimulation of 2',5'-OAS Activity (Median) in Cynomolgus serum after single administration of Soluferon with HSA.

### Literatur

Meyer, Thorsten Ulf, Ph.D. thesis "Expression, Aufreinigung und Charakterisierung einer Variante des humanen Interferon beta aus CHO-Zellen mit verbesserten biochemischen und pharmakokinetischen Eigenschaften" University of Hannover, Germany, October 2000
Mollenkopf et al., 2004, Infect Immun. 72(11): 6471-6479
Nagabhushan & Trotta, Ullmann's Encyclopedia of Industrial Chemistry. A14, VCH, Weinheim, Federal Republic of Germany (1989) 372-374.
Pepinsky RB et al. 2001, "Improved pharmacokinetic properties of a polyethylene glycol-modified form of interferon-beta-1a with preserved in vitro bioactivity", J. Pharmacol. Exp. Therap., 297(3): 1059-1066
"Remington's Pharmaceutical Sciences" (17 ed., 1985).
Smith & Darlington, 1999, "Recent developments in drug therapy for multiple sclerosis.", Mult. Scler. 5(2): 110-120.
Streuli et al., 1980, Science 209: 1343-1349).
Trotta et al. 1987, Developments in Industrial Microbiology 72, Elsevier, Amsterdam: p. 53-64
Ullmann's Encyclopedia of Industrial Chemistry. A14, VCH, Weinheim, Federal Republic of Germany (1989), pp. 372-374
Weissmann 1982, Phil. Trans. R. Soc. Lond. B, 299:7-28

## Claims

1. Type-1 interferon with at least 90%, more preferably at least 98%, most preferred at. least 99% identity to human interferon beta according to SEQ ID No 1 to 4 wherein the amino acids in position 5, 8, 47, 50, 106, 111, 116 and 120 (according to the numbering of SEQ ID No 1 to 4) are substituted with a non-hydrophobic amino acid and the amino acid at position 17 (according to the numbering of SEQ ID No 1 to 4) is substituted by an amino acid other than cysteine and microheterogeneous forms thereof for use in the treatment of multiple sclerosis.

2. Type-1 interferon according to claim 1 wherein the amino acids in position 5, 8, 47, 50, 106, 111, 116 and 120 (according to the numbering of SEQ ID No 1 to 4) are substituted with a amino acid selected from the group of arginine, asparagine, aspartate, glutamate, glutamine, histidine, lysine, methionine, serine, threonine, tryptophane, and tyrosine.

3. Type-1 interferon according to claim 2 wherein the amino acids in position 5, 8, 47, 50, 106, 111, 116 and 120 (according to the numbering of SEQ ID No 1 to 4) are substituted with serine.

4. Type-1 interferon according to any of the above claims wherein the amino acid at position 17 (according to the numbering of SEQ ID No 1 to 4) is substituted with serine.

5. Type-1 interferon according to any of the above claims with a deletion of 1, 2 or 3 amino acid(s) at the N - and/or C-terminus.

6. Type-1 interferon according to any of the above claims wherein the interferon has the amino acid sequence of SEQ: ID No 7.

7. Type-1 interferon according to any of the above claims, wherein the interferon is recombinantly produced.

8. Type-1 interferon according to any of the above claims wherein the patients exhibit a multiple sclerosis selected from the group of relapsing remitting MS, secondary progressive MS, primary progressive MS, progressive relapsing MS, benign MS, Clinically Isolated Syndrome (CIS), Devic's disease, Balo concentric sclerosis, Schilder's diffuse sclerosis and Marburg multiple sclerosis.

9. Pharmaceutical composition comprising a Type-1 interferon according to any of the above claims for use in the treatment of multiple sclerosis.


SEQUENCE LISTING

<110>  Vakzine Project Management GmbH

<120>  Type-1 interferons for treatment of multiple sclerosis

<130>  51 991 K

<160>  7

<170>  PatentIn version 3.3

<210>  1
<211>  166
<212>  PRT
<213>  Homo sapiens

<400>  1

Met Ser Tyr Asn Leu Leu Gly Phe Leu Gln Arg Ser Ser Asn Phe Gln
1               5                   10                  15

Cys Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Leu Gln
        35                  40                  45

Gln Phe Gln Lys Glu Asp Ala Ala Leu Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Leu Glu Lys Glu Asp Phe Thr
            100                 105                 110

Arg Gly Lys Leu Met Ser Ser Leu His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165

<210>  2
<211>  166
<212>  PRT
<213>  Homo sapiens

<400> 2

Met Ser Tyr Asn Leu Leu Gly Phe Leu Gln Arg Ser Ser Asn Phe Gln
1               5                   10                  15

Cys Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Leu Gln
        35                  40                  45

Gln Phe Gln Lys Glu Asp Ala Ala Leu Asn Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Leu Glu Lys Glu Asp Phe Thr
            100                 105                 110

Arg Gly Lys Leu Met Ser Ser Leu His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165

<210>  3
<211>  166
<212>  PRT
<213>  Homo sapiens

<400>  3

Met Ser Tyr Asn Leu Leu Gly Phe Leu Gln Arg Ser Ser Asn Phe Gln
1               5                   10                  15

Cys Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Leu Gln
        35                  40                  45

Gln Phe Gln Lys Glu Asp Ala Ala Leu Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Leu Glu Lys Glu Asp Phe Thr
            100                 105                 110

Arg Gly Lys Leu Met Ser Ser Leu His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Gly Asn
                165

<210>  4
<211>  166
<212>  PRT
<213>  Homo sapiens

<400>  4

Met Ser Tyr Asn Leu Leu Gly Phe Leu Gln Arg Ser Ser Asn Cys Gln
1               5                   10                  15

Cys Gln Lys Leu Leu Trp Gln Leu Asn Gly Arg Leu Glu Tyr Cys Leu
            20                  25                  30

Lys Asp Arg Arg Asn Phe Asp Ile Pro Glu Glu Ile Lys Gln Leu Gln
        35                  40                  45

Gln Phe Gln Lys Glu Asp Ala Ala Val Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Arg Asn
                85                  90                  95

His Leu Lys Thr Val Leu Glu Glu Lys Leu Glu Lys Glu Asp Phe Thr
            100                 105                 110

Arg Gly Lys Arg Met Ser Ser Leu His Leu Lys Arg Tyr Tyr Gly Arg
        115                 120                 125

Ile Leu His Tyr Leu Lys Ala Lys Glu Asp Ser His Cys Ala Trp Thr
    130                 135                 140

Ile Val Arg Val Glu Ile Leu Arg Asn Phe Tyr Val Ile Asn Arg Leu
145                 150                 155                 160

Thr Gly Tyr Leu Arg Asn
                165

<210>  5
<211>  166
<212>  PRT
<213>  Homo sapiens

<220>
<221>  misc_feature
<222>  (5)..(5)
<223>  Xaa can be any naturally occurring amino acid

<220>
<221>  misc_feature
<222>  (8)..(8)
<223>  Xaa can be any naturally occurring amino acid

<220>
<221>  misc_feature
<222>  (17)..(17)
<223>  Xaa can be any naturally occurring amino acid

<220>
<221>  misc_feature
<222>  (47)..(47)
<223>  Xaa can be any naturally occurring amino acid

<220>
<221>  misc_feature
<222>  (50)..(50)
<223>  Xaa can be any naturally occurring amino acid

<220>
<221>  misc_feature
<222>  (106)..(106)
<223>  Xaa can be any naturally occurring amino acid

<220>
<221>  misc_feature
<222>  (111)..(111)
<223>  Xaa can be any naturally occurring amino acid

<220>
<221>  misc_feature
<222>  (116)..(116)
<223>  Xaa can be any naturally occurring amino acid

<220>
<221>  misc_feature
<222>  (120)..(120)
<223>  Xaa can be any naturally occurring amino acid

<400>  5

Met Ser Tyr Asn Xaa Leu Gly Xaa Leu Gln Arg Ser Ser Asn Phe Gln
1               5                   10                  15

Xaa Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Xaa Gln
        35                  40                  45

Gln Xaa Gln Lys Glu Asp Ala Ala Leu Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Xaa Glu Lys Glu Asp Xaa Thr
            100                 105                 110

Arg Gly Lys Xaa Met Ser Ser Xaa His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165

<210>  6
<211>  166
<212>  PRT
<213>  Homo sapiens

<400>  6

Met Ser Tyr Asn Ser Leu Gly Ser Leu Gln Arg Ser Ser Asn Phe Gln
1               5                   10                  15

Ser Gln Lys Leu Leu Trp Gln Leu Asn Gly Arg Leu Glu Tyr Cys Leu
            20                  25                  30

Lys Asp Arg Met Asn Phe Asp Ile Pro Glu Glu Ile Lys Gln Ser Gln
        35                  40                  45

Gln Ser Gln Lys Glu Asp Ala Ala Leu Thr Ile Tyr Glu Met Leu Gln
    50                  55                  60

Asn Ile Phe Ala Ile Phe Arg Gln Asp Ser Ser Ser Thr Gly Trp Asn
65                  70                  75                  80

Glu Thr Ile Val Glu Asn Leu Leu Ala Asn Val Tyr His Gln Ile Asn
                85                  90                  95

His Leu Lys Thr Val Leu Glu Glu Lys Ser Glu Lys Glu Asp Ser Thr
            100                 105                 110

Arg Gly Lys Ser Met Ser Ser Ser His Leu Lys Arg Tyr Tyr Gly Arg
        115                 120                 125

Ile Leu His Tyr Leu Lys Ala Lys Glu Tyr Ser His Cys Ala Trp Thr
    130                 135                 140

Ile Val Arg Val Glu Ile Leu Arg Asn Phe Tyr Phe Ile Asn Arg Leu
145                 150                 155                 160

Thr Gly Tyr Leu Arg Asn
                165

<210>  7
<211>  165
<212>  PRT
<213>  Homo sapiens

<400>  7

Ser Tyr Asn Ser Leu Gly Ser Leu Gln Arg Ser Ser Asn Phe Gln Ser
1               5                   10                  15

Gln Lys Leu Leu Trp Gln Leu Asn Gly Arg Leu Glu Tyr Cys Leu Lys
            20                  25                  30

Asp Arg Met Asn Phe Asp Ile Pro Glu Glu Ile Lys Gln Ser Gln Gln
        35                  40                  45

Ser Gln Lys Glu Asp Ala Ala Leu Thr Ile Tyr Glu Met Leu Gln Asn
    50                  55                  60

Ile Phe Ala Ile Phe Arg Gln Asp Ser Ser Ser Thr Gly Trp Asn Glu
65                  70                  75                  80

Thr Ile Val Glu Asn Leu Leu Ala Asn Val Tyr His Gln Ile Asn His
                85                  90                  95

Leu Lys Thr Val Leu Glu Glu Lys Ser Glu Lys Glu Asp Ser Thr Arg
            100                 105                 110

Gly Lys Ser Met Ser Ser Ser His Leu Lys Arg Tyr Tyr Gly Arg Ile
        115                 120                 125

Leu His Tyr Leu Lys Ala Lys Glu Tyr Ser His Cys Ala Trp Thr Ile
    130                 135                 140

Val Arg Val Glu Ile Leu Arg Asn Phe Tyr Phe Ile Asn Arg Leu Thr

145                 150                 155                 160

Gly Tyr Leu Arg Asn
                165